# EUROPEAN PATENT APPLICATION

(11) **EP 4 803 624 A1**
(43) Date of publication of application: **09.09.2026**
(21) Application number: 24885688.2
(22) Date of filing: 29.10.2024
(51) Int. Cl.: C12N 15/09, C12N 5/10, C12N 5/0789, C12N 15/12

(54) **METHOD FOR PRODUCING HEMATOPOIETIC STEM CELLS IN WHICH BCL11A GENE ENHANCER IS PARTIALLY OR ENTIRELY DESTROYED, AND KIT FOR USE IN THE METHOD**

(30) Priority: 30.10.2023 JP 2023185785
(71) Applicant: C4U Corporation, Suita-shi, Osaka, 565-0871 (JP)
(72) Inventor: KOBORI Shungo, Suita-shi, Osaka 565-0871 (JP); SAEKI Ryota, Suita-shi, Osaka 565-0871 (JP)
(74) Representative: J A Kemp LLP
(86) International application number: PCT/JP2024/038444
(87) International publication number: WO 2025/094917

(57) **Abstract**

A method for producing hematopoietic stem cells in which part or all of an enhancer of the BCL11A gene is disrupted, the method comprising a step of introducing a CRISPR-Cas3 system comprising the following (A) to (C) into hematopoietic stem cells:
(A) a Cas3 protein, a polynucleotide encoding the protein, or an expression vector comprising the polynucleotide,
(B) a Cascade protein, a polynucleotide encoding the protein, or an expression vector comprising the polynucleotide, and
(C) a crRNA targeting an enhancer of the BCL11A gene, a polynucleotide encoding the crRNA, or an expression vector comprising the polynucleotide.

## Description

### [Technical Field]

The present invention relates to a method for producing hematopoietic stem cells in which part or all of an enhancer of the BCL11A gene is disrupted (destroyed), and a kit for use in the method.

### [Background Art]

BCL11A (B-cell lymphoma 11A) is a C2H2-type zinc finger protein and is known to be involved in regulating the expression of various genes. In particular, BCL11A suppresses the expression of γ-globin in adult erythroid progenitor cells and functions as a major transcriptional regulator for the expression switch from γ-globin to β-globin during the transition from fetus to adult. Hemoglobin (Hb) is a tetramer composed of four globin peptides, and the main form of hemoglobin in the fetus is fetal hemoglobin (HbF), which is composed of two α-globins and two γ-globins; however, just before birth, the expression of γ-globin is suppressed by a regulatory mechanism mainly involving BCL11A, and HbF is switched to adult hemoglobin (HbA), which is composed of two α-globins and two β-globins (Haydar Frangoul et al., N Engl J Med, 2021, 384, p. 252-260 (Non Patent Literature 1)). However, even after birth, HbF is present at a proportion of about 1% or less of the total hemoglobin, and because HbF has a high oxygen-carrying capacity, it has been reported, for example, that increasing the expression level of γ-globin, that is, increasing HbF, can alleviate the clinical severity of abnormal hemoglobinopathies such as β-hemoglobinopathy, sickle cell disease (SCD), and β-thalassemia, which are caused by mutations in or decreased expression of β-globin (Non Patent Literature 1; Elenoe C. Smith et al., Blood., 2016, Nov 10; 128 (19): p. 2338-2342 (Non Patent Literature 2)).

For this reason, for example, for the purpose of treating the aforementioned abnormal hemoglobinopathies and the like, Patent Literature 1 (International Publication No. WO2014/085593) describes a method for producing progenitor cells in which the expression of the BCL11A gene is suppressed, and as a method for suppressing the expression of the BCL11A gene, a method of introducing a mutation within 60,716,189-60,728,612 on chromosome 2, the same chromosome as the BCL11A gene, in hematopoietic stem cells is mentioned. Furthermore, for example, Patent Literature 2 (International Publication No. WO2019/113149) describes hematopoietic stem cells in which a mutation has been introduced into the +58 DHS (DNase I hypersensitive site) within the enhancer region of the BCL11A gene. Also, as methods for introducing the mutations, methods of introducing mutations by cleaving the site with a site-specific nuclease such as a zinc finger nuclease, the CRISPR-Cas9 system, or a TALEN are respectively mentioned.

### [Citation List]

### [Patent Literature]

[PTL 1] International Publication No. WO2014/085593
[PTL 2] International Publication No. WO2019/113149

### [Non Patent Literature]

[NPL 1] Haydar Frangoul et al., N Engl J Med, 2021, 384, p. 252-260
[NPL 2] Elenoe C. Smith et al., Blood., 2016, Nov 10; 128 (19): p. 2338-2342

### [Summary of Invention]

### [Technical Problem]

However, when the present inventors investigated the suppression of BCL11A gene expression, they found that even when a mutation is introduced into the enhancer region of the BCL11A gene of hematopoietic stem cells using, for example, the above-described CRISPR-Cas9 system, the suppression of BCL11A gene expression in erythroid progenitor cells differentiated therefrom is not sufficient; as a result, they found that there is a problem in that the release of the suppression of γ-globin expression by BCL11A is incomplete, and the expression level of γ-globin does not sufficiently increase. Also, with the CRISPR-Cas9 system, because the target sequence of the guide RNA is short (usually, preferably about 20 bases), there were also problems of insufficient specificity and the occurrence of off-targets.

The present invention has been made in view of the problems of the prior art described above, and an object is to provide a production method that can highly efficiently produce hematopoietic stem cells capable of differentiating into hematopoietic progenitor cells in which the expression of the BCL11A gene is specifically and sufficiently suppressed, and a kit that can be suitably used in the production method.

### [Solution to Problem]

The present inventors conducted intensive research to achieve the above object and attempted to disrupt the enhancer of the BCL11A gene in hematopoietic stem cells using the CRISPR-Cas3 system. As a result, surprisingly, they found that in erythroid progenitor cells differentiated from hematopoietic stem cells in which the enhancer was disrupted by the CRISPR-Cas3 system, the expression level of γ-globin was significantly increased, despite the genome editing efficiency (deletion rate) at the target site (site to be edited) within the enhancer being low compared to the case of using the conventional CRISPR-Cas9 system. That is, they found that when the CRISPR-Cas3 system was used, the success rate of suppressing BCL11A gene expression relative to the genome editing efficiency at the target site was high compared to when the CRISPR-Cas9 system was used, and that the enhancer of the BCL11A gene can be disrupted more reliably with high specificity and high efficiency. Furthermore, according to the above technology using the CRISPR-Cas3 system, almost no off-targets were confirmed, and they found that the on-target efficiency relative to such a low off-target frequency was also relatively even higher. Therefore, the present inventors found that by using the CRISPR-Cas3 system particularly for disrupting the enhancer of the BCL11A gene, the enhancer of the BCL11A gene can be disrupted with high specificity and high efficiency in hematopoietic stem cells, and as a result, because the expression of the BCL11A gene is sufficiently suppressed in hematopoietic progenitor cells differentiated from the hematopoietic stem cells, the CRISPR-Cas3 system is particularly effective for suppressing BCL11A gene expression, and completed the present invention.

Aspects of the present invention provided based on such findings are as follows.
[1] A method for producing hematopoietic stem cells in which part or all of an enhancer of the BCL11A gene is disrupted, the method comprising a step of introducing a CRISPR-Cas3 system comprising the following (A) to (C) into hematopoietic stem cells:
   (A) a Cas3 protein, a polynucleotide encoding the protein, or an expression vector comprising the polynucleotide,
   (B) a Cascade protein, a polynucleotide encoding the protein, or an expression vector comprising the polynucleotide, and
   (C) a crRNA targeting an enhancer of the BCL11A gene, a polynucleotide encoding the crRNA, or an expression vector comprising the polynucleotide.
[2] The method according to [1], wherein the nucleotide sequence of the crRNA comprises the nucleotide sequence set forth in SEQ ID NO: 27.
[3] The method according to [1] or [2], wherein the produced hematopoietic stem cells are capable of differentiating into erythroid progenitor cells in which the expression level of γ-globin is increased.
[4] A method for improving the expression level of γ-globin in erythroid progenitor cells of a subject, the method comprising a step of administering to the subject hematopoietic stem cells obtained by the method according to any one of [1] to [3].
[5] A method for preventing or treating an abnormal hemoglobinopathy, the method comprising a step of administering to a subject hematopoietic stem cells obtained by the method according to any one of [1] to [3].
[6] A kit for use in the method according to any one of [1] to [5], the kit comprising:
   the following (A) to (C):
   (A) a Cas3 protein, a polynucleotide encoding the protein, or an expression vector comprising the polynucleotide,
   (B) a Cascade protein, a polynucleotide encoding the protein, or an expression vector comprising the polynucleotide, and
   (C) a crRNA targeting an enhancer of the BCL11A gene, a polynucleotide encoding the crRNA, or an expression vector comprising the polynucleotide.

### [Advantageous Effects of Invention]

According to the present invention, it is possible to provide a production method that can highly efficiently produce hematopoietic stem cells capable of differentiating into hematopoietic progenitor cells in which the expression of the BCL11A gene is specifically and sufficiently suppressed, and a kit that can be used in the production method.

When hematopoietic stem cells in which the enhancer of the BCL11A gene is disrupted are differentiated into erythroid progenitor cells, in such erythroid progenitor cells, the expression of the BCL11A gene is suppressed, and because the suppression of expression by BCL11A is released and the expression of γ-globin is induced, the expression level of γ-globin increases. Therefore, the present invention is useful for preventing or treating a group of diseases in which the expression level of γ-globin is involved. Furthermore, according to such a method using the CRISPR-Cas3 system, off-targets can be avoided, making it useful also from the perspective of safety.

### [Brief Description of Drawings]

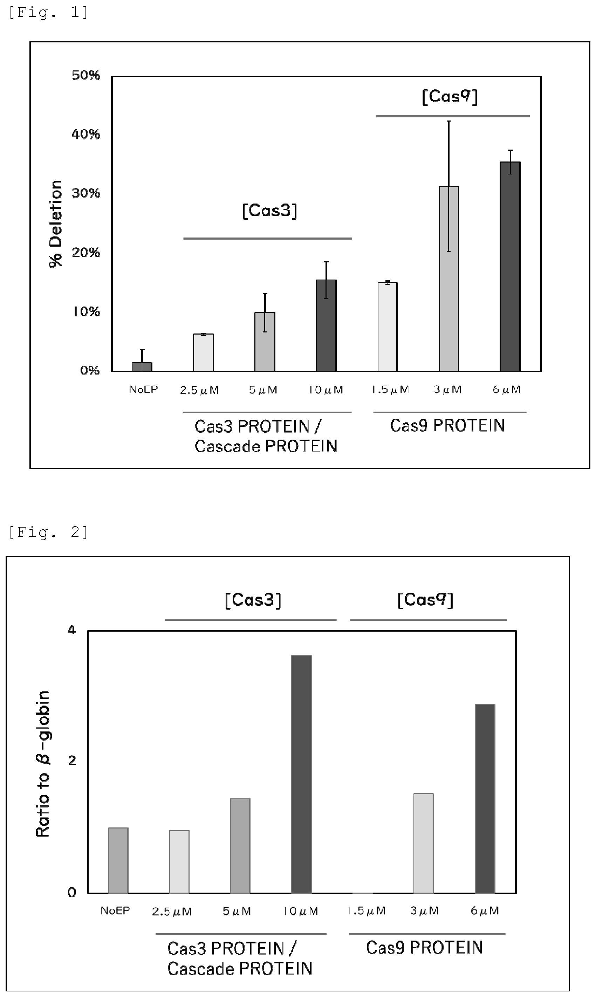

[Fig. 1] is a graph showing, as a result of digital PCR in Test Example 1, the relationship between the introduced amount of Cas3 protein/Cascade protein (Cas3 protein/(Cascade protein complex + crRNA)) or the introduced amount of Cas9 protein and the deletion rate at the target site (% Deletion).
[Fig. 2] is a graph showing, as a result of quantitative PCR in Test Example 1, the relationship between the introduced amount of Cas3 protein/Cascade protein (Cas3 protein/(Cascade protein complex + crRNA)) or the introduced amount of Cas9 protein and the relative expression level of γ-globin (Ratio to β-globin).

### [Description of Embodiments]

Hereinafter, preferred embodiments of the present invention will be described more specifically by way of examples, but the present invention is not limited thereto.

### <Method for Producing Hematopoietic Stem Cells in which Enhancer of BCL11A Gene is Disrupted>

The present invention provides a method for producing hematopoietic stem cells in which part or all of an enhancer of the BCL11A gene is disrupted, the method comprising a step of introducing a CRISPR-Cas3 system comprising the following (A) to (C) into hematopoietic stem cells (in this specification, may be simply referred to as "the production method of the present invention"):
(A) a Cas3 protein, a polynucleotide encoding the protein, or an expression vector comprising the polynucleotide,
(B) a Cascade protein, a polynucleotide encoding the protein, or an expression vector comprising the polynucleotide, and
(C) a crRNA targeting an enhancer of the BCL11A gene, a polynucleotide encoding the crRNA, or an expression vector comprising the polynucleotide.

### (BCL11A Gene, Enhancer of BCL11A Gene, Hematopoietic Stem Cells in which Enhancer of BCL11A Gene is Disrupted)

The "BCL11A gene" is a gene that encodes BCL11A (B-cell lymphoma 11A), which is a C2H2-type zinc finger protein and a subunit of the BAF chromatin remodeling complex (human BCL11A gene reference number (Gene ID): 53335). BCL11A is also referred to as EVI9, CTIP1, DILOS, ZNF856, HBFQTL5, BCL11A-L, BCL11A-S, BCL11a-M, or BCL11A-XL. BCL11A is highly expressed in hematopoietic cells and is known to contribute to the expression switch from γ-globin to β-globin during the transition from fetal hemoglobin (HbF) to adult hemoglobin (HbA). "γ-globin" is a protein encoded by the HBG1 gene and the HBG2 gene in humans. Two γ-globin chains, together with two α-globin chains, constitute fetal hemoglobin (HbF), which is normally replaced by adult hemoglobin (HbA) after birth (Non Patent Literature 1).

The "enhancer of the BCL11A gene" is a region to which a transcription factor capable of promoting transcription of the BCL11A gene binds, and in human genomic DNA, it is known that multiple such regions exist within the introns of the BCL11A gene and upstream or downstream of the BCL11A gene, but the enhancer of the BCL11A gene according to the present invention is preferably an enhancer located within an intron of the BCL11A gene. As the enhancer region of the BCL11A gene according to the present invention, the region of 60,489,054-60,501,476 (12,423 bp) on human chromosome 2 is more preferably mentioned. Also, examples of the transcription factor that bind to the enhancer of the BCL11A gene include GATA, STAT1, ELF1, KLF1, RREB1, and TAL1. For example, "GATA" is a protein belonging to the GATA transcription factor family and is also referred to as GF1, GF-1, NFE1, XLTT, ERYF1, NF-E1, XLANP, XLTDA, and GATA-1. Among the enhancer regions of the BCL11A gene, examples of the region to which GATA binds (GATA binding region) include DHS+55, DHS+58, and DHS+62, which are DNase I hypersensitive sites (DHS) described later.

"Hematopoietic stem cell (HSC)" is a cell that has the ability to self-replicate and differentiate into progenitor cells of hematopoietic lineage cells (in this specification, may be referred to as "hematopoietic progenitor cells"). Examples of the hematopoietic lineage cells include leukocytes, erythrocytes, platelets, mast cells, dendritic cells, eosinophils, neutrophils, monocytes, macrophages, granulocytes, T cells, B cells, and NK cells, and examples of the hematopoietic progenitor cells include leukocyte progenitor cells, erythroid progenitor cells, megakaryocyte progenitor cells, granulocyte progenitor cells, pro-B cells, pro-T cells, and pro-NK cells. It is preferable for the hematopoietic stem cells used in the production method of the present invention to be differentiated into erythroid progenitor cells, which are cells that produce γ-globin, from the viewpoint that, for example, the expression level of γ-globin can be improved as a result of the treatment method of the present invention described below. In the present invention, "differentiation of hematopoietic stem cells into hematopoietic progenitor cells (preferably erythroid progenitor cells)" means not only that the hematopoietic stem cells themselves differentiate into the progenitor cells, but also encompasses that one or more hematopoietic stem cells produced by self-replication of said hematopoietic stem cells each differentiate into the progenitor cells to produce one or more progenitor cells.

Hematopoietic stem cells exhibit many known phenotypes, for example, CD34+, CD38-. The hematopoietic stem cells used in the production method of the present invention can be isolated from, for example, peripheral blood, bone marrow, umbilical cord, placenta, etc., by a conventionally known method or a method analogous thereto, using the aforementioned phenotypes as an index.

The origin of the hematopoietic stem cells according to the present invention includes animals, preferably humans or non-human mammals, and examples of the non-human mammals include artiodactyls such as cattle, wild boars, pigs, sheep, and goats; perissodactyls such as horses; rodents such as mice, rats, guinea pigs, hamsters, and squirrels; rabbits, dogs, cats, and ferrets. Among these, when administering hematopoietic stem cells produced by the production method of the present invention to a subject, it is preferable that the hematopoietic stem cells used in the production method of the present invention are from the same animal as the subject. Also, the hematopoietic stem cells used in the production method of the present invention may be cultured cells (for example, stem cells such as induced pluripotent stem (iPS) cells) derived from cells constituting an animal, cells constituting organs/tissues extracted from an animal, or the like, in addition to cells collected from peripheral blood, bone marrow, umbilical cord, placenta, etc. of these animals.

The production method of the present invention produces hematopoietic stem cells in which the enhancer of the BCL11A gene is disrupted. In the present invention, the simple term "disruption of an enhancer" includes not only the total disruption of the enhancer but also partial disruption. The "hematopoietic stem cells in which part or all of an enhancer of the BCL11A gene is disrupted" obtained by the production method of the present invention means that they are cells in which part or all of the enhancer of the BCL11A gene is deleted or substituted (preferably deleted) and its enhancer function is suppressed. "Suppression of the enhancer function of the BCL11A gene" more specifically indicates that, for example, at least one of the aforementioned transcription factors, preferably at least one or more GATA, does not bind.

The site to be deleted or substituted in the enhancer of the BCL11A gene is preferably at least part or all of the region of 60,489,054-60,501,476 on human chromosome 2. The site to be deleted or substituted (preferably deleted) in the enhancer of the BCL11A gene is particularly preferably part or all of the aforementioned GATA binding region, and more specifically, it is preferably at least any one of the following three regions: DHS+55 (60,498,289-60,498,552 (264 bp) on human chromosome 2), DHS+58 (60,495,103-60,495,330 (228 bp) on human chromosome 2), and DHS+62 (60,490,907-60,491,050 (144 bp) on human chromosome 2); it is more preferable that it is part (for example, 75% or more) or all of two of these regions, and even all three regions, respectively; and it is still more preferable that it is all of all three regions, respectively.

Confirmation that part or all of the enhancer of the BCL11A gene is disrupted can be performed based on a conventionally known method, and can be confirmed by, for example, a PCR method (digital PCR method, real-time PCR method, endpoint PCR method, etc.), a sequencing method, a Southern blotting method, a next-generation sequencing method, etc.

### (CRISPR-Cas3 System)

CRISPR-Cas systems are divided into Class 1, which cleaves a target region (region to be cleaved) with a complex of multiple proteins, and Class 2, which cleaves with a single protein. The CRISPR-Cas9 system, CRISPR-Cas12 (Cpf1) system, CRISPR-Cas13 system, etc., which have been developed as genome editing tools so far, are all classified as Class 2, but the CRISPR-Cas3 system according to the present invention belongs to Type I of Class 1. The Type I system is classified into multiple subtypes, and is currently classified into Type I-A, I-B, I-C, I-D, I-E, and I-F, and Type I-G, which is a subtype of Type I-B (see, for example, [van der Oost J et al., Nature Reviews Microbiology, 2014, Vol. 12 (No. 7), p. 479-492], [Jackson RN et al., Current Opinion in Structural Biology, 2014, Vol. 24, p. 106-114], [Makarova et al., Nat Rev Microbiol., 2020, 18, p. 67-83]).

The "CRISPR-Cas3 system" according to the present invention specifically comprises the following (A) to (C):
(A) a Cas3 protein, a polynucleotide encoding the protein, or an expression vector comprising the polynucleotide,
(B) a Cascade protein, a polynucleotide encoding the protein, or an expression vector comprising the polynucleotide, and
(C) a crRNA targeting an enhancer of the BCL11A gene, a polynucleotide encoding the crRNA, or an expression vector comprising the polynucleotide.

### [Cas Protein Group]

The Cas3 protein constituting the CRISPR-Cas3 system has DNA cleavage activity and helicase activity. The Cas3 protein can cleave a target DNA at multiple sites by cooperating with the Cascade protein and crRNA that constitute the CRISPR-Cas3 system. In this specification, the simple description "Cas3" shall mean "Cas3 protein." Also, in this specification, the Cas3 protein and the Cascade protein are collectively referred to as the "Cas protein group" in some cases.

The CRISPR-Cas3 system according to the present invention encompasses all seven subtypes of Type I. The Type I-E system, which is common among Type I CRISPR-Cas3 systems, includes Cas3, Cas8 (Cse1), Cas11 (Cse2), Cas5, Cas6, and Cas7 as the Cas protein group, and this Cas protein group cleaves the target DNA by cooperating with the crRNA. In this case, the Cas protein group typically forms a complex (Cascade complex) of one molecule of Cas3, one molecule of Cas8, two molecules of Cas11, six molecules of Cas7, one molecule of Cas5, and one molecule of Cas6.

In the Type I-A system, the Cas protein group includes Cas3-HD, Cas3-HEL, Cas5, Cas6, Cas7, Cas8, and Cas11; in the Type I-B system, the Cas protein group includes Cas3, Cas5, Cas6, Cas7, Cas8, and Cas11; in the Type I-C system, the Cas protein group includes Cas3, Cas5, Cas7, Cas8, and Cas11; in the Type I-D system, the Cas protein group includes Cas3, Cas5, Cas6, Cas7, Cas10, and Cas11; in the Type I-F system, the Cas protein group includes Cas2-3, Cas5, Cas6, Cas7, and Cas8; and in the Type I-G system, the Cas protein group includes Csb2 (Cas6-like), Cas7, Cas8g, Cas3, and Cas11. However, regarding Cas11, even when it is excluded from the components of the Type I system, each system exhibits DNA cleavage activity (for example, it is known that in Type I-B and Type I-C systems, even without Cas11, DNA cleavage activity is exhibited, albeit at a lower level compared to when Cas11 is included, and the same is true for the Type I-D system). Therefore, the CRISPR-Cas3 system according to the present invention also includes systems that do not include Cas11.

The origin of the Cas protein group according to the present invention is not particularly limited, but from the viewpoint of being suitable for genome editing in animal cells, an Escherichia coli origin is preferable. The amino acid sequence of each protein constituting the Cas protein group according to the present invention can be obtained from, for example, a public database (Genbank, etc.), but as one preferred embodiment of the constitution of the Cas protein group, as sequences of a typical E. coli-derived Type I-E system, the following constitution is mentioned:
Cas3: a protein comprising the amino acid sequence set forth in SEQ ID NO: 1
Cas8 (Cse1): a protein comprising the amino acid sequence set forth in SEQ ID NO: 3
Cas11 (Cse2): a protein comprising the amino acid sequence set forth in SEQ ID NO: 5
Cas5: a protein comprising the amino acid sequence set forth in SEQ ID NO: 7
Cas6: a protein comprising the amino acid sequence set forth in SEQ ID NO: 9
Cas7: a protein comprising the amino acid sequence set forth in SEQ ID NO: 11.

Also, the Cas protein group according to the present invention includes variants that have occurred naturally or have been artificially modified. Therefore, as another preferred embodiment of the constitution of the Cas protein group according to the present invention, a constitution in which each protein is a protein comprising an amino acid sequence having high identity with each amino acid sequence of the above typical Cas protein group, and in which Cas3 has DNA cleavage activity (and further preferably the Cas protein group has Cascade complex formation ability), is mentioned. High identity is, for example, an amino acid sequence identity of 80% or more, preferably 85% or more, more preferably 90% or more (for example, 91% or more, 92% or more, 93% or more, 94% or more), and still more preferably 95% or more (for example, 96% or more, 97% or more, 98% or more, 99% or more). The amino acid sequence identity can be determined using, for example, BLAST (Basic Local Alignment Search Tool at the National Center for Biological Information) or the like (for example, using default, i.e., initial setting parameters).

As another preferred embodiment of the constitution of the Cas protein group according to the present invention, a constitution in which each protein is a protein comprising an amino acid sequence in which one or more amino acids are substituted, deleted, added, and/or inserted in each amino acid sequence of the above typical Cas protein group, and in which Cas3 has DNA cleavage activity (and further preferably the Cas protein group has Cascade complex formation ability), is also mentioned. Here, "multiple" is usually within 50 amino acids, preferably within 30 amino acids, more preferably within 20 amino acids, and particularly preferably within 10 amino acids (for example, within 5 amino acids, within 3 amino acids, within 2 amino acids, 1 amino acid).

"Having DNA cleavage activity" indicates that it can cleave a DNA strand in at least one location. That the Cas3 has DNA cleavage activity and Cascade complex formation ability can be confirmed, for example, by showing DNA cleavage activity that is equal to or greater than (for example, 50% or more, 80% or more) that when the above typical Cas protein group is used.

A functional molecule may be further added to each of the proteins constituting the Cas protein group, as necessary. Examples of the functional molecule include a nuclear localization signal for promoting migration into the nucleus of a eukaryotic cell (for example, as described in Wu J et al., 2009, Biophysical journal, Vol. 96 (Issue 9), p. 3840-3849, etc.), a tag for facilitating purification (for example, an HN tag, a His tag, a FLAG tag, a glutathione-S-transferase (GST) tag), a reporter protein for facilitating detection (for example, a fluorescent protein such as green fluorescent protein (GFP), a chemiluminescent protein such as luciferase), and one of these or a combination of two or more thereof may be used, but it is not limited to these. When adding the functional molecule, it can be added to, for example, the N-terminal side and/or the C-terminal side of each protein.

Hereinafter, the Type I-E CRISPR-Cas3 system will be described as a representative example, but for other types of CRISPR-Cas3 systems, the Cascade proteins constituting the system may be read as appropriate.

In the CRISPR-Cas3 system according to the present invention, each protein constituting the Cas protein group can be independently introduced into the hematopoietic stem cells in the form of a protein, in the form of a polynucleotide encoding the protein, or in the form of an expression vector comprising the polynucleotide.

When introducing the Cas protein group into cells in the form of proteins, it is possible to appropriately prepare the amounts and the like of each protein, which is preferable from the viewpoint of handling and from the viewpoint that DNA is not introduced into the genome.

Each protein constituting such a Cas protein group can be prepared by a known method or a method analogous thereto. For example, as a method for preparing the Cas3 protein, the method described in International Publication No. WO2022/186063 is mentioned. Also, the Cascade proteins can be prepared, for example, by the method described in Kazuto Yoshimi et al., Nature Communications, 2022, 13:4917, https://doi.org/10.1038/s41467-022-32618-0 (hereinafter referred to as "Reference I").

When introducing the Cas protein group into cells in the form of proteins, it is preferable to introduce into the cells a pre-formed complex comprising two or more of the proteins constituting the Cas protein group, from the viewpoint of intracellular cleavage efficiency and the like. Examples of combinations of proteins in the complex to be introduced into the cells include a complex consisting of two molecules of Cas11; a complex consisting of six molecules of Cas7; a complex consisting of one molecule of Cas8, one molecule of Cas5, and one molecule of Cas6; a complex consisting of one molecule of Cas3, one molecule of Cas8, two molecules of Cas11, six molecules of Cas7, one molecule of Cas5, and one molecule of Cas6; and a combination of one or two or more of these. Furthermore, as the Cas protein group, it is more preferable to introduce into the cells a pre-formed complex of the aforementioned complex and the crRNA described below. Such a complex can be prepared by a known method or a method analogous thereto, and can be prepared, for example, by the method described in the above Reference I.

When introducing the Cas protein group into cells in the form of a polynucleotide, the polynucleotide may consist only of DNA, or may consist of RNA, GNA, LNA, BNA, PNA, TNA, etc., or a mixture thereof. It may also be modified by components other than nucleic acids, such as sugar chains. Such a polynucleotide can be prepared by a known method or a method analogous thereto, and can be, for example, artificially synthesized.

When introducing the Cas protein group into cells in the form of an expression vector, the expression vector is preferably one that can stably express the encoded protein without being integrated into the host genome. As a base vector for such an expression vector, various commonly used vectors can be used, and can be appropriately selected according to the cells to be introduced, the introduction method, etc. Examples of such base vectors include phage vectors, plasmid vectors, viral vectors, retroviral vectors, chromosomal vectors, episomal vectors, and vectors derived from viruses (bacterial plasmids, bacteriophages, yeast episomes, etc.), yeast chromosomal elements and viruses (baculoviruses, papovaviruses, vaccinia viruses, adenoviruses, fowlpox viruses, pseudorabies viruses, herpesviruses, lentiviruses, retroviruses, etc.), and vectors derived from a combination of two or more of these (cosmids, phagemids, etc.).

The expression vector preferably further includes sites for transcription initiation and transcription termination, and includes a ribosome binding site in the transcription region. The expression vector also preferably includes one or two or more of a promoter sequence corresponding to the type of cell to be introduced, a sequence for enhancing transcription from DNA (for example, an enhancer sequence), and a sequence for stabilizing the transcribed RNA (for example, a polyA addition sequence). Furthermore, in the expression vector, the polynucleotides encoding each protein constituting the Cas protein group may each be independently codon-optimized as appropriate.

When introducing multiple proteins constituting the Cas protein group into cells in the form of an expression vector, multiple polynucleotides respectively encoding the multiple proteins may be included in the same expression vector, and the number thereof is not particularly limited as long as the function of the CRISPR-Cas3 system can be exerted in the host cell into which the expression vector has been introduced. For example, it is possible to design it such that all polynucleotides encoding each protein constituting the Cas protein group are loaded onto one type of (the same) expression vector, and it is also possible to design it such that some or one of the polynucleotides encoding each protein constituting the Cas protein group are loaded onto separate expression vectors. For example, it is possible to design it such that the polynucleotides encoding the Cascade proteins are loaded onto one type of (the same) expression vector, and the polynucleotide encoding Cas3 is loaded onto another expression vector. From the viewpoint of expression efficiency and the like, a method of loading the polynucleotides encoding each protein constituting the Cas protein group onto six separate types of expression vectors is preferable. In addition, for the purpose of adjusting the expression level, etc., multiple polynucleotides encoding the same protein may be loaded onto the same expression vector. For example, it is possible to design it such that the polynucleotide encoding Cas3 is placed in two locations within one type of (the same) expression vector. Also, when the expression vector includes multiple polynucleotides respectively encoding multiple proteins constituting the Cas protein group, a nucleotide sequence encoding an amino acid sequence that is cleaved by an intracellular protease (such as a 2A peptide) may be inserted between the multiple polynucleotides.

Such an expression vector can be prepared by a known method or a method analogous thereto. For example, in addition to the methods described in the instruction manuals attached to kits for vector construction, methods described in various handbooks, for example, the method described in "Joseph Sambrook & David W. Russell, Molecular cloning: a laboratory manual 3rd Ed., New York: Cold Spring Harbor Laboratory Press, 2001," can be adopted.

### [crRNA]

The crRNA (CRISPR RNA) constituting the CRISPR-Cas3 system has a nucleotide sequence complementary to a target sequence on a target DNA. The crRNA according to the present invention targets the enhancer region of the BCL11A gene as the target region for cleavage (cleavage target region), and in humans, the target DNA is chromosome 2 where the enhancer of the BCL11A gene is present, and the cleavage target region is within the enhancer region of the BCL11A gene (preferably, within the region of at least one of DHS+55, DHS+58, and DHS+62). The target sequence can be selected according to the cleavage target region, and more specifically, it is preferably within the range of 60,485,907-60,503,552 on human chromosome 2, preferably within a range of 5,000 bp before and after the cleavage target region (enhancer region of the BCL11A gene, preferably at least one of DHS+55, DHS+58, and DHS+62), and more preferably includes, for example, 60,495,486-60,495,517 on human chromosome 2. Also, in general, the length of the target sequence is preferably 32-37 bases (Ming Li et al., Nucleic Acids Res., 2017, May 5; 45(8): p. 4642-4654).

In the CRISPR-Cas3 system according to the present invention, the crRNA can be introduced into the hematopoietic stem cells in the form of RNA, in the form of a polynucleotide encoding the RNA, or in the form of an expression vector comprising the polynucleotide.

As a CRISPR-Cas3 system, when introducing crRNA into the eukaryotic hematopoietic stem cells in the form of RNA alone, separate from the Cas protein group, or in the form of a polynucleotide encoding the crRNA or an expression vector comprising it, the crRNA is preferably the pre-crRNA described in International Publication No. WO2018/225858. In the present invention, "pre-crRNA" has a structure in which a repeat sequence, etc. is added to the crRNA that functions as a component of the CRISPR-Cas3 system in the cell (may be referred to as "mature crRNA"), and typically has a "leader sequence-repeat sequence-spacer sequence-repeat sequence (LRSR structure)" or "repeat sequence-spacer sequence-repeat sequence (RSR structure)" structure. Typical embodiments of the leader sequence and repeat sequence are as described in International Publication No. WO2018/225858. The pre-crRNA becomes a mature crRNA when cleaved by a Cascade protein (for example, Cas6 in Type I-A, B, D-E, Cas5 in Type I-C).

A crRNA (mature crRNA) typically has a nucleotide sequence complementary to the target sequence and a 3' terminal repeat sequence (one unit is about 10-70 bases, preferably 30-50 bases, including at least one hairpin structure), and the hairpin structure and one or more proteins constituting the Cas protein group (typically Cas6) interact to form a complex. For this reason, the crRNA recognizes the target sequence and binds to the target DNA, guides the Cas protein group that forms a complex with the crRNA to the vicinity of the cleavage target region, and the guided Cas protein group exposes a single-stranded DNA by the helicase activity of Cas3 and cleaves the cleavage target region by the DNA cleavage activity.

In the enhancer of the BCL11A gene (cleavage target region), cleavage by the CRISPR-Cas3 system occurs at a position determined by both the complementarity of base pairing between the nucleotide sequence of the crRNA and the target sequence, and the PAM sequence present on the 5' side of the complementary strand of the target sequence.

The PAM sequence for the CRISPR-Cas3 system according to the present invention is "AAG" or a similar base sequence (for example, "AGG," "GAG," "TAC," "ATG," "TAG," etc.) adjacent to the 5' side of the target sequence.

The sequence of the crRNA (including mature crRNA and pre-crRNA) may be appropriately designed according to the target sequence. As one preferred embodiment of the crRNA according to the present invention, for example, a sequence comprising a nucleotide sequence complementary to the target sequence (60,495,486-60,495,517 of human chromosome 2) is mentioned, and more specifically, a sequence comprising the nucleotide sequence set forth in SEQ ID NO: 27 is mentioned.

When introducing the crRNA into a cell in the form of RNA or in the form of a polynucleotide encoding the RNA, these polynucleotides can be prepared by a known method or a method analogous thereto, and can be, for example, artificially synthesized.

Also, when introducing the crRNA into a cell in the form of an expression vector, the expression vector can be prepared by a known method or a method analogous thereto, and for example, those described for the Cas protein group as its expression vector, including their preferred embodiments, can be appropriately adopted.

### (Introduction Step)

In the production method of the present invention, the CRISPR-Cas3 system is introduced into the hematopoietic stem cells, and by the DNA cleavage activity of Cas3 guided to the vicinity of the cleavage target region on the enhancer of the BCL11A gene via the binding of the target region and the crRNA, the inside of the enhancer of the BCL11A gene is cleaved (preferably cleaved at multiple sites), thereby disrupting part or all of the enhancer of the BCL11A gene.

As described above, the method for introducing the CRISPR-Cas3 system into the hematopoietic stem cells may be a method of introducing the CRISPR-Cas3 system in the form of a protein or RNA, or a method of introducing a polynucleotide encoding these or an expression vector comprising the polynucleotide and expressing it in the cell. Therefore, as the CRISPR-Cas3 system, each protein constituting the Cas protein group may be independently introduced into the cell in the form of a protein, or introduced into the cell in the form of RNA or DNA (polynucleotide) encoding the protein and expressed in the cell, or introduced into the cell in the form of a vector that expresses the protein (expression vector) and expressed in the cell. Also, independently of this, the crRNA may be introduced into the cell in the form of RNA (polynucleotide), or introduced into the cell in the form of DNA (polynucleotide) encoding the RNA and expressed in the cell, or introduced into the cell in the form of a vector that expresses the RNA (expression vector) and expressed in the cell.

When introducing the CRISPR-Cas3 system into a cell in the form of an expression vector and expressing it in the cell, for example, a vector that expresses each protein constituting the Cas protein group and a vector that expresses the crRNA may be introduced into the cell separately, or a vector that expresses a combination of two or more of these may be introduced into the cell.

The method for introducing the CRISPR-Cas3 system according to the present invention into a cell is not particularly limited, and a known method for introducing proteins, DNA fragments, RNA fragments, and vectors into cells can be appropriately adopted according to the cell type, etc. Examples of such methods include electroporation, microinjection, particle gun method, calcium phosphate method, polyethyleneimine (PEI) method, liposome method (lipofection), DEAE-dextran method, cationic lipid-mediated transfection, viruses (adenovirus, lentivirus, adeno-associated virus, baculovirus, etc.), Agrobacterium method, lithium acetate method, spheroplast method, heat shock method (calcium chloride method, rubidium chloride method), etc. Such methods are described in many standard laboratory manuals, such as "Davis et al., Basic methods in molecular biology, New York: Elsevier, 1986."

When the CRISPR-Cas3 system is introduced into a cell (including expression within the cell), the CRISPR-Cas3 system and the enhancer of the BCL11A gene (cleavage target region) come into contact via the binding of the target region and the crRNA, and by the cleavage of the cleavage target region by the DNA cleavage activity of the CRISPR-Cas3 system described above, part or all of the enhancer of the BCL11A gene is disrupted (preferably deleted).

### (Use of Hematopoietic Stem Cells in which Enhancer of BCL11A Gene is Disrupted)

The production method of the present invention can also be expressed as a method for producing hematopoietic stem cells capable of differentiating into erythroid progenitor cells in which the expression of the BCL11A gene is suppressed, or a method for producing hematopoietic stem cells capable of differentiating into erythroid progenitor cells in which the expression of γ-globin is promoted. That is, when the hematopoietic stem cells obtained by the production method of the present invention are differentiated into erythroid progenitor cells, because the expression of their BCL11A gene is suppressed by the disruption of the enhancer, the expression of γ-globin, which is normally suppressed by BCL11A in adults, is promoted. In the present invention, "expression" includes both expression at the transcription level (the process by which DNA is transcribed into mRNA) and expression at the translation level (the process by which mRNA is translated into a peptide, polypeptide, or protein), and also includes the step of splicing mRNA, but when referring to "expression of the BCL11A gene," it mainly means expression at the transcription level.

Therefore, for example, by administering the hematopoietic stem cells obtained by the production method of the present invention to a subject, when the hematopoietic stem cells differentiate into erythroid progenitor cells in the living body of the subject, the expression level of γ-globin in the erythroid progenitor cells of the subject can be increased. An increase in the expression level of γ-globin and the resulting increase in fetal hemoglobin (HbF) contribute to the prevention and treatment of abnormal hemoglobinopathies such as β-hemoglobinopathy, sickle cell disease (SCD), and β-thalassemia (Non Patent Literature 1, Non Patent Literature 2).

Therefore, the present invention also provides a method for improving the expression level of γ-globin in erythroid progenitor cells of a subject, comprising a step of administering to the subject hematopoietic stem cells obtained by the production method of the present invention, and a method for preventing or treating an abnormal hemoglobinopathy, comprising a step of administering to a subject hematopoietic stem cells obtained by the production method of the present invention (in this specification, these may be collectively referred to as "the treatment method of the present invention").

Examples of the subject include the animals mentioned above, with humans being preferable. Examples of the administration method include injection and transplantation.

### <Kit>

The present invention provides a kit for use in the production method of the present invention and the treatment method of the present invention, the kit comprising:
the following (A) to (C):
(A) a Cas3 protein, a polynucleotide encoding the protein, or an expression vector comprising the polynucleotide,
(B) a Cascade protein, a polynucleotide encoding the protein, or an expression vector comprising the polynucleotide, and
(C) a crRNA targeting an enhancer of the BCL11A gene, a polynucleotide encoding the crRNA, or an expression vector comprising the polynucleotide.

The above (A) to (C), including their preferred embodiments, are each as described above in the CRISPR-Cas3 system of the production method of the present invention. Each of these may independently be in the form of a protein or RNA, in the form of a polynucleotide encoding the protein or RNA, or in the form of an expression vector comprising the polynucleotide and expressing the protein or RNA. These forms, including their preferred embodiments, are also each as described above.

Also, when (C) is in the form of an expression vector, the expression vector may be
(C') an expression vector comprising an insertion site for a polynucleotide encoding a crRNA that targets an enhancer of the BCL11A gene,
so that the user can design the crRNA according to the target sequence. In this case, the insertion site may be configured to insert only the nucleotide sequence complementary to the target sequence among the polynucleotides encoding the crRNA, and the other part of the polynucleotide encoding the crRNA may be included in the expression vector in advance. When (C) is in the form of a polynucleotide or an expression vector encoding a crRNA, the crRNA is preferably the above-mentioned pre-crRNA.

In the kit of the present invention, the combination of forms of (A) to (C) is not particularly limited, and it may be a combination product individually comprising (A) to (C), or a pre-mixed product of two or more of (A) to (C), and may also be a composition comprising these and other components (solvent, etc.). For example, (B) may be in a different form for each Cascade protein among the above, in the form of a complex or composition comprising two or more Cascade proteins, or in the form of a polynucleotide respectively encoding two or more Cascade proteins, or an expression vector comprising the polynucleotide. Also, (A) and (B) together may be in the form of a complex or composition comprising multiple proteins constituting the Cas protein group, or in the form of a polynucleotide respectively encoding two or more proteins, or an expression vector comprising the polynucleotide. Furthermore, for example, when introducing (A) and (B) as proteins and (C) as RNA into a cell, they may be in the form of a pre-formed complex of two or more of these, or when introducing (A) to (C) into a cell in the form of an expression vector, they may be in the form of an expression vector comprising two or more polynucleotides.

The kit of the present invention may further comprise one or more additional reagents. Examples of such additional reagents include reagents for separating hematopoietic stem cells, culture medium for culturing hematopoietic stem cells, dilution buffers, reconstitution solutions, washing buffers, nucleic acid introduction reagents, protein introduction reagents, and control reagents, but are not limited to these. The kit may also further comprise instructions for carrying out the production method of the present invention and the treatment method of the present invention.

Also, when the kit of the present invention is used in the treatment method of the present invention, it may further be provided with, for example, pharmaceutical additives and equipment for administering the produced hematopoietic stem cells as a pharmaceutical composition to a subject. The pharmaceutical composition can be prepared by a conventional method. More specifically, it can be prepared by formulating the hematopoietic stem cells produced by the production method of the present invention with pharmaceutical additives, for example, according to the administration method. Examples of the pharmaceutical additives include excipients, binders, disintegrants, lubricants, fluidizers (anti-caking agents), colorants, plasticizers, solvents, solubilizing agents, emulsifiers, suspending agents (adhesives), thickeners, pH adjusters (acidifying agents, alkalizing agents, buffering agents), wetting agents (solubilizers), antimicrobial preservatives, chelating agents, medical water, stabilizers, preservatives, etc., and one or two or more of these may be used.

Each element included in the kit may be contained in a separate container, or may be contained in the same container. Each element may be contained in a container for each single use amount, or multiple use amounts may be contained in one container. Each element may be contained in a container in a dry form, or may be contained in a container in a form dissolved in a suitable solvent (a solvent containing a buffer, a stabilizer, a preservative, an antiseptic, etc.).

### [Examples]

Hereinafter, the present invention will be described more specifically based on examples, but the present invention is not limited to the following examples.

### <Test Example 1> Disruption of the Enhancer of the BCL11A Gene in Human Hematopoietic Stem Cells by the CRISPR-Cas3 System

### (1) Enhancer of the BCL11A Gene

The cleavage target region within the enhancer of the BCL11A gene to be disrupted in this test example was set around 60,495,269 of chromosome 2 (chr2) of human genomic DNA.

### (2) CRISPR-Cas System

### (CRISPR-Cas3 System (Example))

The CRISPR-Cas3 system used in this test example was constructed. The amino acid sequence of the Cas3 protein and the nucleotide sequence encoding it are shown in SEQ ID NO: 1 and SEQ ID NO: 2, respectively. The Cas3 protein was prepared and purified by the method described in International Publication No. WO2022/186063.

The Cascade protein consists of Cas8 (Cse1)-Cas11 (Cse2)-Cas7-Cas5-Cas6, and was a Cascade protein complex consisting of one molecule of Cas8, two molecules of Cas11, six molecules of Cas7, one molecule of Cas5, and one molecule of Cas6. The amino acid sequences of the Cas8 protein, Cas11 protein, Cas5 protein, Cas6 protein, and Cas7 protein and the nucleotide sequences encoding them are shown in SEQ ID NO: 3 to 12, respectively, in order. Each Cascade protein and the Cascade protein complex were prepared and purified by the method described in Reference I.

The crRNA targeting the enhancer of the BCL11A gene was synthesized with 60,495,486-60,495,517 of chromosome 2 (chr2) of human genomic DNA as the target sequence. Table 1 below shows the target sequence (underlined) and the PAM sequence (bold) of the crRNA. The crRNA was prepared and purified as a mature crRNA by the method described in Reference I. Also, the Cas3 protein, the Cascade protein complex, and the crRNA were formed into a protein-RNA complex by the method described in Reference I, and this was used as the CRISPR-Cas3 system.

### (CRISPR-Cas9 System (Comparative Example))

As a comparative subject, a CRISPR-Cas9 system was constructed. The Cas9 protein was obtained from Integrated DNA Technologies, Inc. Also, the gRNA was synthesized with 60,495,264-60,495,283 of chromosome 2 (chr2) of human genomic DNA as the target sequence. Table 1 below shows the target sequence (underlined) and the PAM sequence (bold) of the gRNA.

**[Table 1]**

| Nucleotide sequence (5'-3') | SEQ ID NO |
|---|---|
| crRNA target sequence (underlined) and PAM sequence (bold) | 13 |
| **aag**tcctgtctagctgccttccttatcacaggaat | |
| gRNA target sequence (underlined) and PAM sequence (bold) | 14 |
| ctaacagttgcttttatcac**agg** | |

### (3) Cell Culture

As human hematopoietic stem cells (hHSCs), human CD34+ cells derived from umbilical cord blood of healthy individuals obtained from StemExpress, Folsom CA were used. The hHSCs were first cultured in a medium (StemSpan SFEM, Veritas Corporation, to which StemSpan(TM) CD34+ Expansion Supplement (10x) was added). Then, on day 4 from the start of culture, the CRISPR-Cas systems constructed in (2) above were respectively introduced by electroporation, and the cells were cultured under the same conditions until day 7 from the start of culture (3 days after introducing the CRISPR-Cas system).

Then, on day 7 from the start of culture, to induce differentiation of hHSCs into erythroid progenitor cells, the medium was first replaced with a medium of the following composition: StemSpan SFEM (Veritas Corporation), 50 ng/mL SCF, 20 ng/mL EPO, 10 ng/mL IL-3, 10 ng/mL IGF-1, 1 µM dexamethasone, and cultured for 5 days (until day 12 from the start of culture). Then, on day 12 from the start of culture, the medium was replaced with a medium of the following composition: StemSpan SFEM (Veritas Corporation), 20 ng/mL EPO, 10 ng/mL IGF-1, 0.5 mg/mL human-transferrin, 2% BSA, to induce differentiation, and the cells were further cultured for 6 days (until day 18 from the start of culture) to obtain erythroid progenitor cells.

### (4) Electroporation

The electroporation in (3) above was performed on a cell population of one million cells containing hHSCs on day 4 from the start of culture by an electroporation method (Maxcyte-Atx (MaxCyte, Inc.), program: HSC-5). Each CRISPR-Cas system constructed in (2) above was introduced into the cell population to have the following composition:

### (CRISPR-Cas3 system)

Protein-RNA complex: Cas3 protein/Cascade protein: Cas3 protein/(Cascade protein complex + crRNA) (Cas3 protein:(Cascade protein complex + crRNA) = 1:1 (molar ratio)): 2.5 µM, 5 µM, or 10 µM

### (CRISPR-Cas9 system)

Cas9 protein: 1.5 µM, 3 µM, or 6 µM
gRNA: 6 µM.

### (5) Digital PCR

In (3) above, on day 3 after introducing each CRISPR-Cas system (day 7 from the start of culture), genomic DNA was collected from the cells, and the proportion of deletion at the target site within the enhancer of the BCL11A gene was determined by digital PCR. In this test, region A (60,495,229-60,495,399 on human chromosome 2: 171 bp), which includes part of DHS+58, a GATA binding region, was used as the point for determining the presence or absence of genome editing (target site), and when this region A was not amplified by PCR, it was judged that "the target site within the enhancer of the BCL11A gene was deleted." The DNeasy Blood & Tissue Kit (Qiagen) was used for genome extraction, and the QIAcuity (Qiagen) was used for digital PCR. As a comparative subject, unedited cells (NoEP) cultured under the same conditions without introducing any of the CRISPR-Cas systems constructed in (2) above were used. Also, each deletion rate was calculated as the deletion rate (% Deletion) by the following formula: (1 - (amount of amplification product of region A in CRISPR-Cas system-introduced cells / amount of amplification product of internal control in CRISPR-Cas system-introduced cells) / (amount of amplification product of region A in NoEP / amount of amplification product of internal control in NoEP)) x 100, using a region near the BCL11A gene that is not cleaved by the CRISPR-Cas system as an internal control. Table 2 below shows the nucleotide sequences of each primer and probe used for digital PCR. In Table 2, HEX/BHQ1 and FAM/BHQ1 respectively indicate a combination of a reporter dye and a quencher dye, and each amplification product amount was taken as the fluorescence intensity derived from the reporter dye.

**[Table 2]**

| Probe/Primer | | Nucleotide sequence (5'-3') | SEQ ID NO |
|---|---|---|---|
| For target site | Target_probe (fluorescent) | ttatcacaggctccaggaaggg (HEX/BHQ1) | 15 |
| | Target_Fwd | tgattccagtgcaaagtcca | 16 |
| | Target_Rev | caccctaatcagaggccaaa | 17 |
| For internal control | Reference_probe (fluorescent) | tagatgtggcaagtcagaccacag (FAM/BHQ1) | 18 |
| | Reference_Fwd | tgaagttttccaggggtttg | 19 |
| | Reference_Rev | ctttctctgagggcatggag | 20 |

### (6) Quantitative PCR (qPCR)

After (3) above, on day 18 from the start of culture, mRNA was collected from the cells, and after reverse transcription reaction, the expression levels (mRNA amounts) of γ-globin and β-globin were calculated by quantitative PCR. As a comparative subject, unedited cells (NoEP) cultured under the same conditions without introducing any of the CRISPR-Cas systems constructed in (2) above were used. Also, each expression level was calculated using the ACTB gene as an internal control (normalize), and the relative expression level of γ-globin expression level to β-globin expression level (Ratio to β-globin) was determined for each. Table 3 below shows the nucleotide sequences of each primer used for quantitative PCR.

**[Table 3]**

| Primer | | Nucleotide sequence (5'-3') | SEQ ID NO |
|---|---|---|---|
| For γ-globin | γ-globin_Fwd | cttccttgggagatgccat | 21 |
| | γ-globin_Rev | gaattctttgccgaaatggat | 22 |
| For β-globin | β-globin_Fwd | ctttagtgatggcctggct | 23 |
| | β-globin_Rev | cactggtggggtgaattct | 24 |
| For internal control | ACTB_Fwd | caccattggcaatgagcggttc | 25 |
| | ACTB_Rev | aggtctttgcggatgtccacgt | 26 |

As a result of the digital PCR in (5) above, the relationship between the introduced amount of Cas3 protein/Cascade protein (Cas3 protein/(Cascade protein complex + crRNA)) or the introduced amount of Cas9 protein and the deletion rate at the target site (% Deletion) is shown in Fig. 1. Also, as a result of the quantitative PCR in (6) above, the relationship between the introduced amount of Cas3 protein/Cascade protein (Cas3 protein/(Cascade protein complex + crRNA)) or the introduced amount of Cas9 protein and the relative expression level of γ-globin (Ratio to β-globin) is shown in Fig. 2. Furthermore, the relationship between the deletion rate and the relative expression level of γ-globin when the introduced amount of Cas3 protein/Cascade protein (Cas3 protein/(Cascade protein complex + crRNA)) was 10 µM and the introduced amount of Cas9 protein was 6 µM is shown in Table 4 below.

**[Table 4]**

| CRISPR-Cas system | | (a) Deletion rate (%) | (b) γ-globin relative expression level | (b)/(a) |
|---|---|---|---|---|
| CRISPR-Cas3 | Cas3 protein: 5µM / Cascade protein: 5µM | 16 | 3.6 | 0.225 |
| CRISPR-Cas9 | Cas9 protein: 6µM / gRNA: 6µM | 35 | 2.9 | 0.083 |

As shown in Fig. 1, the proportion of deletion at the target site within the enhancer of the BCL11A gene by the CRISPR-Cas3 system was 16% (10 µM), whereas the proportion of deletion at the target site within the enhancer of the BCL11A gene by the CRISPR-Cas9 system was 35% (6 µM), and the deletion rate at the target site, that is, the genome editing efficiency, was higher when the CRISPR-Cas9 system was used. On the other hand, as shown in Fig. 2 and Table 4, the γ-globin expression level increased to 3.6 times that of the unedited cells (10 µM) by the CRISPR-Cas3 system, whereas the amount of increase by the CRISPR-Cas9 system was 2.9 times (6 µM), and the expression level of γ-globin was significantly increased when the CRISPR-Cas3 system was used. Also, as shown in Table 4, the relative expression level of γ-globin relative to the deletion rate at the target site within the enhancer of the BCL11A gene ((b)/(a)) was about 3 times greater when the CRISPR-Cas3 system was used.

In this way, although the genome editing efficiency (proportion of deletion at the target site) was higher when the CRISPR-Cas9 system was used, the amount of increase in γ-globin expression was much higher when the CRISPR-Cas3 system was used, and it was confirmed that the expression of γ-globin could be induced with high efficiency. Therefore, it was confirmed that by the method using the CRISPR-Cas3 system of the present invention, the enhancer of the BCL11A gene can be disrupted with high specificity and high efficiency in hematopoietic stem cells, and as a result, the expression of the BCL11A gene can be sufficiently suppressed in differentiated hematopoietic progenitor cells.

### <Test Example 2> Analysis of Genome Deletion Patterns by CRISPR-Cas System

### (1) Analysis of Genome Deletion Patterns by CRISPR-Cas9 System

Among the genomic DNA collected on day 3 after introducing the CRISPR-Cas9 system into cells in <Test Example 1> (day 7 from the start of culture), using a sample judged as "the target site within the enhancer of the BCL11A gene was deleted" by the digital PCR of <Test Example 1> (5) as a template, region B (60,494,994-60,495,500 on human chromosome 2: 507 bp) containing DHS+58, a GATA binding region, was amplified by PCR. Then, for the amplified fragment, Sanger sequencing was performed using a sequencing primer (60,495,481-60,495,500 on human chromosome 2: 20 bp). The obtained Sanger sequencing waveform data (.ab1) was analyzed with SYNTHEGO ICE Analysis (Synthego Corporation) to obtain a genome deletion pattern. As a comparative subject, analysis was similarly performed using genomic DNA similarly collected from unedited cells (NoEP) cultured under the same conditions without introducing any of the CRISPR-Cas systems.

### (2) Analysis of Genome Deletion Patterns by CRISPR-Cas3 System

### (2-1) Preparation of NGS Library

An Oligo panel (Twist Bioscience) for enriching the region containing DHS+58, a GATA binding region, was designed for region C (60,493,189-60,543,189 bp on human chromosome 2). Among the genomic DNA collected on day 3 after introducing the CRISPR-Cas3 system into cells in <Test Example 1> (day 7 from the start of culture), region C was enriched from a sample judged as "the target site within the enhancer of the BCL11A gene was deleted" by the digital PCR of <Test Example 1> (5), using the Twist Library Prep EF Kit 2.0, Twist UMI Adapter System TruSeq Compatible, Twist Std Hyb and Wash Kit, Twist Dry Down Beads, Twist Universal Blockers, and the above Oligo panel (all from Twist BioScience), and a library for analyzing the deletion pattern of the enhancer of the BCL11A gene was constructed. The constructed NGS library was analyzed with a Nova-seq (Illumina, Inc.). As a comparative subject, a library was similarly prepared using genomic DNA similarly collected from unedited cells (NoEP) cultured under the same conditions without introducing any of the CRISPR-Cas systems.

### (2-2) Analysis of Genome Deletion Patterns by CRISPR-Cas3 System

For the raw NGS data obtained in (2-1) above, after Quality Check and genome mapping, Split reads (meaning Structure variants) were extracted. From the extracted Split reads, the genome deletion length of the region containing DHS+58, a GATA binding region, by the CRISPR-Cas3 system was analyzed.

As a result of the analyses in (1) and (2) above, whereas in the genome deletion pattern by the CRISPR-Cas9 system, only deletions of several bases (1-20 bases) were confirmed, in the genome deletion pattern by the CRISPR-Cas3 system, a wide range of deletions spanning several kilobases (49-20,699 bases) were confirmed. That is, among the enhancer regions of the BCL11A gene, there are multiple regions to which GATA binds (GATA binding regions) (DHS+55, DHS+58, DHS+62), but whereas with the CRISPR-Cas9 system, the range that can be deleted with a single system was limited to part of a single GATA binding region, according to the method using the CRISPR-Cas3 system of the present invention, it was confirmed that multiple GATA binding regions can be deleted with a single system, and the enhancer of the BCL11A gene can be disrupted more reliably.

### <Test Example 3> Analysis of Off-Targets by CRISPR-Cas3 System

### (1) Extraction of Off-Target Candidates for crRNA

Gene sequences and oncogene sequences with high homology to the base sequence of the crRNA prepared in <Test Example 1> (2), which targeted 60,495,486-60,495,517 of chromosome 2 (chr2) of human genomic DNA, were extracted from the human genome and human oncogenes by in silico analysis, and 92 loci, obtained by removing duplicates from the top 50 loci each, were designated as off-target candidate loci.

### (2) Preparation of NGS Library

An Oligo panel was designed for each of the 92 off-target candidate loci extracted in (1) above, enriched in the same manner as in <Test Example 2> (2-1), and from a sample judged as "the target site within the enhancer of the BCL11A gene was deleted" by the digital PCR of <Test Example 1> (5) among the genomic DNA collected on day 3 after introducing the CRISPR-Cas3 system into cells in <Test Example 1> (day 7 from the start of culture), an NGS library for off-target analysis was constructed. At the time of this library construction, a deletion (1 kb deletion) fragment of a human endogenous genomic region was added as a control for genome cleavage detection at a ratio of 1% to the amount of genomic DNA of each sample. The constructed NGS library was analyzed with a Nova-seq 6000 (Illumina, Inc.).

### (3) In Silico Analysis of Off-Targets by CRISPR-Cas3 System

For the raw NGS data obtained in (2) above, after Quality Check and genome mapping, Split reads of the off-target candidate loci were extracted. As a comparative subject, Split reads were similarly extracted by analyzing in the same manner genomic DNA collected from unedited cells (NoEP) cultured under the same conditions without introducing any of the CRISPR-Cas systems, and the numbers of signals were compared. The cleavage at each off-target candidate locus was evaluated using the number of detected reads of the deletion fragment of the human endogenous genomic region added to each sample as an index for when 1% genome editing efficiency was detected.

As a result of the above analysis, cleavage was not detected at any of the off-target candidate loci, and it was confirmed that according to the disruption of the enhancer of the BCL11A gene using the CRISPR-Cas3 system of the present invention, the induction of off-target cleavage is sufficiently suppressed.

### [Industrial Applicability]

As described above, according to the present invention, it is possible to provide a production method that can highly efficiently produce hematopoietic stem cells capable of differentiating into hematopoietic progenitor cells in which the expression of the BCL11A gene is specifically and sufficiently suppressed, and a kit that can be suitably used in the production method.

When hematopoietic stem cells in which the enhancer of the BCL11A gene is disrupted are differentiated into erythroid progenitor cells, in such erythroid progenitor cells, the expression of the BCL11A gene is suppressed, and because the suppression of expression by BCL11A is released and the expression of γ-globin is induced, the expression level of γ-globin increases. Therefore, the present invention is useful for preventing or treating a group of diseases in which the expression level of γ-globin is involved. Furthermore, according to such a method using the CRISPR-Cas3 system, off-targets can be avoided, making it useful also from the perspective of safety.

## Claims

1. A method for producing hematopoietic stem cells in which part or all of an enhancer of the BCL11A gene is disrupted, the method comprising a step of introducing a CRISPR-Cas3 system comprising the following (A) to (C) into hematopoietic stem cells:
(A) a Cas3 protein, a polynucleotide encoding the protein, or an expression vector comprising the polynucleotide,
(B) a Cascade protein, a polynucleotide encoding the protein, or an expression vector comprising the polynucleotide, and
(C) a crRNA targeting an enhancer of the BCL11A gene, a polynucleotide encoding the crRNA, or an expression vector comprising the polynucleotide.

2. The method according to claim 1, wherein the nucleotide sequence of the crRNA comprises the nucleotide sequence set forth in SEQ ID NO: 27.

3. The method according to claim 1, wherein the produced hematopoietic stem cells are capable of differentiating into erythroid progenitor cells in which the expression level of γ-globin is improved.

4. A method for improving the expression level of γ-globin in erythroid progenitor cells of a subject, the method comprising a step of administering to the subject hematopoietic stem cells obtained by the method according to claim 1.

5. A method for preventing or treating an abnormal hemoglobinopathy, the method comprising a step of administering to a subject hematopoietic stem cells obtained by the method according to claim 1.

6. A kit for use in the method according to any one of claims 1 to 5, the kit comprising:
the following (A) to (C):
(A) a Cas3 protein, a polynucleotide encoding the protein, or an expression vector comprising the polynucleotide,
(B) a Cascade protein, a polynucleotide encoding the protein, or an expression vector comprising the polynucleotide, and
(C) a crRNA targeting an enhancer of the BCL11A gene, a polynucleotide encoding the crRNA, or an expression vector comprising the polynucleotide.
